# EUROPEAN PATENT APPLICATION

(11) **EP 3 939 507 A1**
(43) Date of publication of application: **19.01.2022**
(21) Application number: 20186112.7
(22) Date of filing: 16.07.2020
(51) Int. Cl.: A61B 5/16, A61B 5/00, A61B 5/145, A61B 5/055

(54) **EMDR THERAPY ANALYSIS**

(71) Applicant: Koninklijke Philips N.V., 5656 AG Eindhoven (NL)
(72) Inventor: MENA BENITO, Maria Estrella, 5656 AE Eindhoven (NL); WESTERINK, Joanne Henriëtte Desirée Monique, 5656 AE Eindhoven (NL); VAN EE, Raymond, 5656 AE Eindhoven (NL); LEUFKENS, Timmy Robertus Maria, 5656 AE Eindhoven (NL)
(74) Representative: Philips Intellectual Property & Standards

(57) **Abstract**

Two subsequent fMRI images taken during or around an EMDR therapy treatment are compared. A change in a trauma level metric in relevant brain anatomy of a traumatized patient is determined and communicated, such that the success of the EMDR therapy can be more objectively assessed and/or the information may be used to tailor the EMDR therapy a during or after a therapy session.

## Description

### FIELD OF THE INVENTION

The present invention generally relates to an EMDR therapy analysis device and method, as well as a computer program product.

### BACKGROUND OF THE INVENTION

Post-traumatic stress disorder (PTSD) is a serious mental health condition that comes as a result of the brain's inability to adequately process an extremely psychologically distressing event. Eye Movement Desensitization and Reprocessing therapy (EMDR) is a technique used to treat PTSD. EMDR is also applied for the treatment of many other traumatic experiences. The EMDR technique involves a unique procedure in which a therapist exposes the patient to bilateral stimulation (BLS), which induces repetitive (horizontal) eye movements, while the patient thinks of the traumatic experience. The effort needed for the eye movements helps the patient to reprocess the experience and become less sensitive for it.

A problem with current EMDR therapy is that it makes use of a standard method for all patients. Furthermore, nowadays the approach utilizes non-consistent and non-objective criteria for evaluation of recovery and treatment strategies. Also, the effectiveness of the treatment usually can only be determined after the therapy has ended.

### SUMMARY OF THE INVENTION

It is one object of the present invention to tailor EMDR strategies for an optimal and personalized therapeutic outcome for patients with a psychological or mental trauma.

Embodiments according to the present invention are directed to an Eye Movement Desensitization and Reprocessing (EMDR) therapy analysis device, and a corresponding method and computer program, for analyzing an EMDR therapy for treating a patient with a psychological or mental trauma. The device comprises a computer implemented system which comprises a computer processor. The computer processor is configured to receive first functional magnetic resonance imaging (fMRI) data from a patient before or during undergoing an EMDR treatment, wherein the first fMRI data comprises fMRI data from an area of interest in the patient's brain or spinal cord that is known to be associated with a trauma level of the patient. The processor is further configured to receive second fMRI data from the patient during or after undergoing an EMDR treatment, wherein the second fMRI data is obtained after the first fMRI data and comprises fMRI data from the area of interest in the patient's brain or spinal cord that is known to be associated with the patient's trauma. The processor is further configured to analyze the first fMRI data and the second fMRI data by identifying a trauma level metric indicating trauma level of the patient and to compare the trauma level metric of the second fMRI data with the first fMRI data and determine whether the trauma level of the patient has changed. And, the computer processor is configured to communicate whether the trauma level has changed. As such the device is able to analyze an EMDR therapy session and provide a patient-specific indication of the effectiveness of the therapy and communicate the result, for instance to the therapist, to a further processor or to an EMDR device.

In a preferred embodiment the computer processor is further configured to receive third updated fMRI data from a patient during or after undergoing an EMDR treatment, wherein the third fMRI data is obtained after the second fMRI data and comprises fMRI data from the area of interest in the patient's brain or spinal cord that is known to be associated with the patient's trauma; analyze the first updated fMRI data and the second updated fMRI data by identifying a trauma level metric indicating trauma level of the patient; compare the trauma level metric of the third updated fMRI data with the second fMRI data and determine whether the trauma level of the patient has changed. As such, the device can now provide an indication of the effectiveness of the treatment of three (or more) moments during or around the treatment and can provide more or more precise effectiveness information, e.g. form before, during and after the sessions.

In a preferred embodiment the computer processor is further configured to receive a sequence of at least two consecutive updated fMRI data from a patient during or after undergoing an EMDR treatment, wherein the fMRI data comprises fMRI data from the area of interest in the patient's brain or spinal cord hat is known to be associated with the patient's trauma; analyze at least two updated fMRI data by identifying a trauma level metric indicating trauma level of the patient, wherein the at least two updated fMRI data comprises an earlier obtained MRI data and a later obtained fMRI data, which was obtained at a later stage than the earlier fMRI data, preferably the later fMRI data is the last obtained fMRI data of the sequence of consecutive fMRI data; compare the trauma level metric of the later fMRI data with the earlier fMRI data and determine whether the trauma level of the patient has changed. As such the effectiveness of the treatment may be determined and communicated at various points and at multiple moments during or around the session or even at later stages after a therapy session.

In a preferred embodiment the first fMRI data is data from the patient before undergoing the EMDR treatment. As such, a baseline value of brain (area) activity may be obtained.

In a preferred embodiment the last obtained fMRI data is data from the patient after undergoing the EMDR treatment. This allows to determine the effectiveness of the treatment directly or at some time after the EMDR treatment session.

In a preferred embodiment the trauma metric of interest is a blood oxygenation level dependent (BOLD) contrast. This is a particularly useful, well-studied and often used metric in fMRI.

In a preferred embodiment wherein the trauma is post-traumatic stress disorder (PTSD) and the area of interest in the patient's brain preferably comprises the patient's amygdala, preferably the patient's left amygdala, and/or the patient's prefrontal cortices. EMDR has been proven to be particularly useful for treating PTSD. An effective EMDR treatment can be particularly well determined from fMRI images of the patient's amygdala and/or prefrontal cortices.

In a preferred embodiment the trauma level is further determined by combining received EMDR data with other physiologic or biometric data of the patient, for instance skin conductance, electrical activity of the brain, heart rate, breath intensity, eye blinking rate, body temperature or any other physical property of the patient that could serve as an indication of effectiveness and/or other fMRI or fMRI-like data, such as regional cerebral blood flow (CBF), cerebral blood volume (CBV), cerebral metabolic rate of oxygen (CMRO2) or local brain conductivity (for instance as obtained by electric properties tomography). As such the effectiveness parameter may be determined even more precise by taking into account other data that may be a measure of the EMDR therapy having a (temporary or lasting) effect. The different data may be part of the communication form the claimed device or may be communicated separately. The therapist or a (deep learning) algorithm may analyze the communicated data causing an adaptation or continuation of the therapy.

In a preferred embodiment the computer processor is configured to communicate whether the trauma level has changed to a communication device that is available to an EMDR therapist during a therapy, for instance by displaying visual information or through auditory or tactile signals, preferably during or shortly after an EMDR therapy session. This allows the therapist to be informed about the effectiveness of the therapy while it is being applied or shortly after, allowing for immediate adaptation of the therapy or deciding if further sessions are necessary.

In a preferred embodiment the computer processor is configured to communicate whether the trauma level has changed to a device that is available to a device that is capable of adapting the EMDR therapy during an ongoing EMDR therapy session, such as a device for generating adaptive visual stimulation, such as an MDR device comprising a light tube (23) and/or pulsators (231), and/or providing a suggestion or plan for a future EMDR session for the patient.

In a preferred embodiment the processor is further configured to translate a trauma level metric change into an effectiveness parameter, for instance a known value of the trauma level metric and/or a relative value compared to an initial value, for instance a relative value, e.g. a percentage, of the value of the trauma level metric of the first fMRI data; and wherein the communication device is further configured to communicate the effectiveness parameter. This is a particular effective way of determining, and optionally quantifying or standardizing, the effectiveness of the EMDR treatment.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Fig. 1 shows a schematic cross section of a human brain and illustrates the position of several brain anatomies mentioned in this document.
Fig. 2 shows a schematic depiction of how an EMDR therapy may be performed by a therapist (Fig. 2a,b) and with the use of an exemplary EMDR device (Fig. 2c).
Fig. 3 illustrates an example of a BOLD response following a neural activity.
Fig. 4 depicts an illustrative EMDR session of a patient in an MR imaging device (Fig. 4a) and a patient in a head coil within the MR imaging device (Fig. 4b).
Fig. 5 depicts constructed illustrative examples of output from a device and method of the present invention as claimed of a fictional traumatized patient treated in one (Fig. 5a) or more (Fig. 5b) EMDR treatment sessions.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESRIPTION OF EMBODIMENTS

In order to contextualize the claimed invention more details about PTSD and EMDR are provided.

PTSD may develop after a person is exposed to a traumatic event, such as sexual assault, warfare, traffic collisions, child abuse, or other threats on a person's life. PTSD develops as a result of the brain being unable to adequately process a traumatic event. When the event is extremely intense, it may overwhelm the human body's processing systems and a person may have a difficult time coping with the emotional distress. As a result, disturbing thoughts and emotions surrounding the incident can persist long after the trauma has occurred.

Symptoms may include disturbing thoughts, feelings, or dreams related to the events, mental or physical distress to trauma-related cues, attempts to avoid trauma-related cues, alterations in how a person thinks and feels, and an increase in the fight-or-flight response. Other signs of PTSD may include reliving the original incident through flashbacks or trauma; feelings of sadness or anger; feeling detached or estranged from people; and having strong negative responses to things as simple as an accidental touch or loud noise. These symptoms last for more than a month after the event and may be present for years if not treated and may cause the patient discomfort, reduced enjoyment of life and/or even a danger to themselves or others.

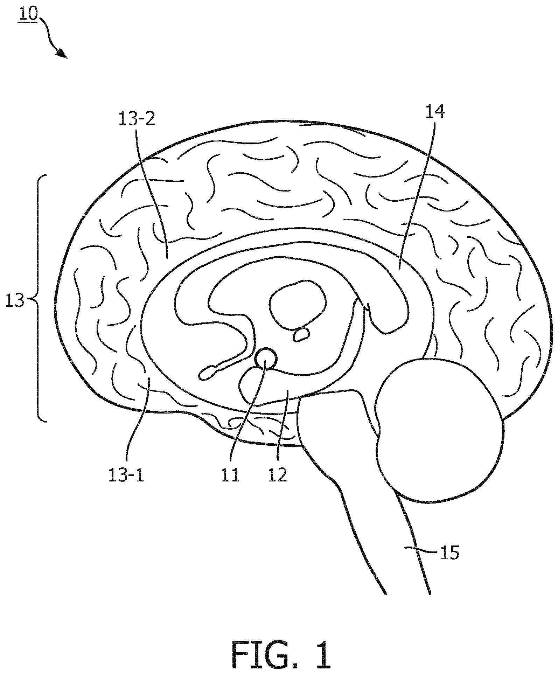

People with PTSD have decreased brain activity in the dorsal and rostral anterior cingulate cortices 14 and the ventromedial prefrontal cortex 13-1 of the brain 10, areas linked to the experience and regulation of emotion (see Fig. 1). The amygdala 11 is strongly involved in forming emotional memories, especially fear-related memories. During high stress, the hippocampus 12, which is associated with placing memories in the correct context of space and time and memory recall, is suppressed. This suppression may be the cause of the flashbacks that can affect people with PTSD. When someone with PTSD undergoes stimuli similar to the traumatic event, the body perceives the event as occurring again because the memory was never properly recorded in the person's memory. The amygdalocentric model of PTSD proposes that the amygdala 11 is very much aroused and insufficiently controlled by the medial prefrontal cortex 13-2 and the hippocampus 12, in particular during extinction. This is consistent with an interpretation of PTSD as a syndrome of deficient extinction ability. The basolateral nucleus (BLA) of the amygdala 11 is responsible for the comparison and development of associations between unconditioned and conditioned responses to stimuli, which results in the fear conditioning present in PTSD.

Eye Movement Desensitization and Reprocessing therapy (EMDR) is a well-researched therapy and one recommended by the World Health Organization (WHO) to treat trauma. EMDR is based on the idea that our brain naturally wants to heal and achieve optimal mental health, and that trauma can overwhelm our natural ability to heal and become "stuck" in our memory. Associations around the traumatic incident remain negative and destructive, and patients may become sensitive to people, places, or things that remind us of the event. As it has proven to be a very effective therapy, psychotherapists mainly use EMDR to treat PTSD patients.

EMDR uses so-called adaptive resolution to transform the trauma from something that has the potential to be distressing and triggering, into a form that is no longer associated with negative, charged emotional, physical, behavioral, and cognitive responses. This is the 'desensitization and reprocessing' component of EMDR. The key to adaptive resolution is relearning, so that the harmful and disturbing information related to the trauma (again, thoughts, feelings and behaviors) are replaced with new, more "adaptive" information and associations.

The standard EMDR protocol comprises two main stages, desensitization of traumatic memories and development and installation of a "resource", such as safe thoughts. The latter is called resource development and installation (RDI). In the standard protocol, both stages use alternating bilateral stimulation (BLS). BLS is performed concurrently with the recall of the worst image of the trauma and the resources installation.

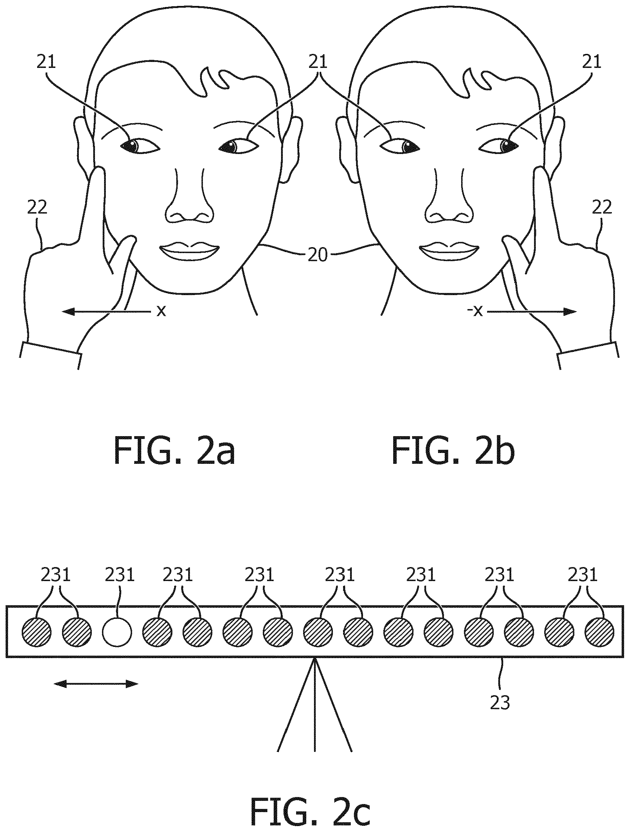

EMDR technique involves a unique procedure in which a therapist exposes the patient 20 to BLS, which involves alternating bilateral visual, eye movement. This stimulus could be their hands, fingers or a stick 22 which is moved x, -x in front of the patient's face and which the patient 20 must follow with their eyes 21 (see Fig. 2a, b). In a more modern set-up EMDR equipment may be in the form of a device for providing visual stimulation, for instance a device comprising a light tube 23 and pulsators 231 is used, in which the pulsators 231, e.g. LEDs, switch on and off sequentially to form moving light path for the patient 20 to follow see Fig. 2c. Alternative versions of this EMDR device may make use of changes in luminance, color, etc. or provide alternate elements or a grid of pulsators instead of a row. The bilateral stimulation could be also auditory or other sensory stimulation e.g. tactile stimulation.

During EMDR therapy, clinical observations suggest that an accelerated learning process is stimulated by standardized EMDR procedures that incorporate use of eye movements. While clients briefly focus on the trauma memory and simultaneously experience BLS, the vividness and emotion of the memory are reduced.

Traditionally, EMDR is carried out by manual guidance, the therapist guides the patient's eye 21 movements with the finger or hand 22. One limitation of EMDR is that manual rate and position controlling of the movements is inaccurate and the speed is inconsistent, even for experienced EMDR therapists, which affect the result of the treatment. In some cases EMDR therapy is provided by the aforementioned EMDR equipment using a light tube 23 and pulsators 231that can be adjusted by the therapist.

Another limitation of the current EMDR therapy is that it uses a standardized 'one-size-fits-all' method for treating PTSD for all patients, and is not adapted to specific characteristics of the patient (20).

A further limitation of traditional EMDR therapy is that it requires a well-trained and experienced EMDR therapist. During the therapy the therapist needs to check the therapeutic effect with the patient. This is done by asking questions regarding sensations in order to adjust the motion of the finger or the motion of light in EMDR equipment. This requires a strong multitasking performance by the therapist and strong focus of the patient on both the auditory and visual information. This could result in that in some cases the therapy does not reach the desirable therapeutic effect.

An additional shortcoming of traditional EMDR therapy is that the progress of a treatment session and subsequently the number of sessions needed is based on the personal (subjective) evaluation of the therapist. That is, the therapist relies on the patient's answers, responses during the procedure and patient's description of their symptoms before and after the EMDR procedure.

Also, afterwards it is not always easy to determine the cause of an unsuccessful session or the most effective part of a successful session.

From the previous, it is clear that there is a need for dedicated, more objective and effective tailoring of EMDR treatment and sessions for best therapeutic outcome and follow-up decision for an individual patient. It is therefore the object of this invention to provide a standardized, but personalized, and controllable EMDR therapy.

One insight underlying the presently claimed invention is that it would be beneficial to eliminate or at least reduce human variables from evaluating the patient in real-time during the procedure and potentially have quantitative or qualitative data of the patient's brain response to the EMDR treatment. It may seem counterintuitive to remove a human component in a therapy such as EMDR (particularly in the field of psychiatric treatment where personal interpretation of information by the therapist is traditionally highly valued). Especially EMDR relies strongly upon interaction between a therapist and a patient, but the presently claimed invention does not replace human interaction but provides an additional tool to improve the session or its follow-up, which still requires a professional therapist for auditory and/or visual guidance, as well as monitoring the patient's response. In preferred embodiments of the present invention the therapist remains in control of the decisions to maintain or adapt the EMDR therapy.

A further insight is that functional magnetic resonance imaging (fMRI) is an imaging technique that would be particularly suitable for this purpose.

In fMRI brain activity is measured by detecting changes associated with blood flow. This technique relies on the fact that cerebral blood flow and neuronal activation are coupled. When an area of the brain is in use, blood flow to that region increases.

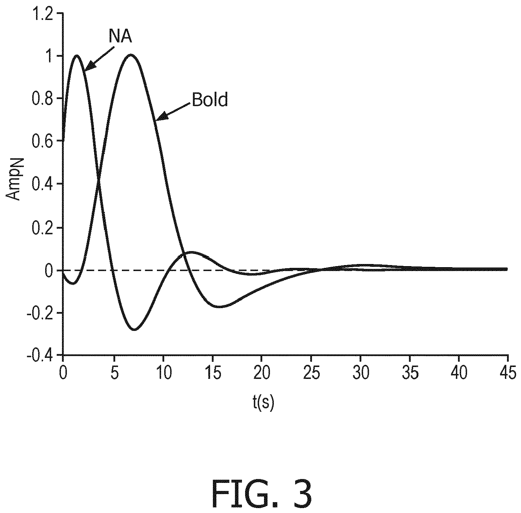

In fMRI a magnetic resonance image, or a spatial or temporal series thereof, is obtained from a subject's brain 10 or spinal cord 15 from which brain activity can be determined because blood flow in the brain is directly related to brain activity. When an area of the brain is active, blood flow to that area increases. The main property that is usually measured by fMRI is blood-oxygen-level dependent (BOLD) contrast, in which neural activity is determined by imaging changes in blood flow (hemodynamic response) in specific areas of the brain or spinal cord. Fig. 3 shows an exemplary graph in which the (normalized) amplitude Amp_{N} is tracked over time (t) for neural activity (NA) and the (delayed) response of the BOLD signal (BOLD). Other fMRI techniques are also available, but BOLD is the most common and well-studied fMRI method.

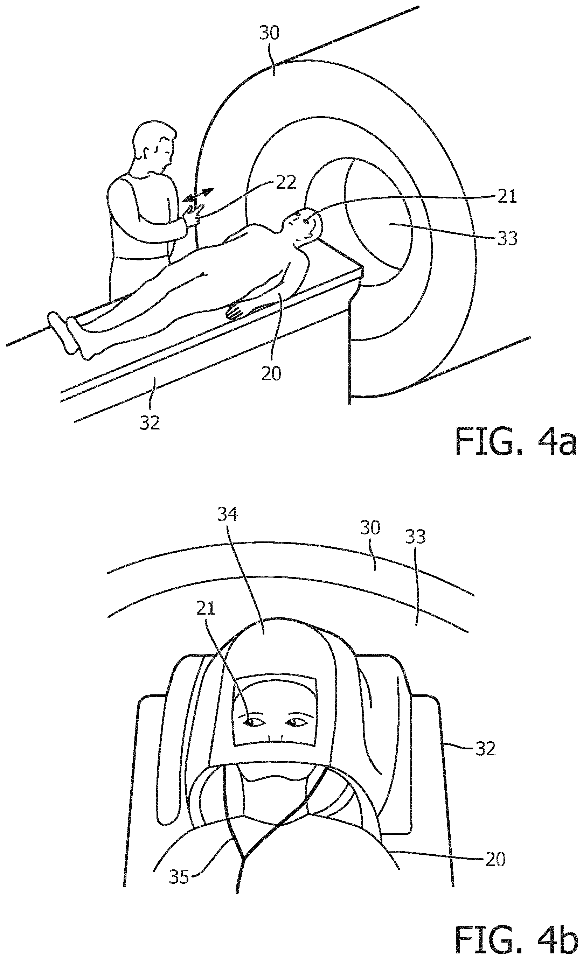

Fig. 4a shows an exemplary drawing of a traumatized patient 20 in the bore 33 of an MRI device 30 undergoing an EMDR therapy while being scanned. In this embodiment the EMDR therapist stands beside the patient support 32 and the patient's eye 21 follow the hand signals 22 as in a normal EMDR therapy session. Fig. 4a is an extremely simplified drawing. In reality the patient's head is covered for a large part by a head coil (32) (see Fig. 4b) and due to the loud sounds generated by the MRI device 30 the patient would not be able to hear the therapist, so sound may be provided through MR-compatible earphones 35. As the patient's view is strongly restricted the therapist must either be positioned such that his hands 22 are in view of the patient or it may be indirectly shown to the patient through a mirror or MR-compatible display system (not shown). Alternatively (MR-compatible) EMDR equipment comprising a light tube (23) and pulsators (231), similar to the one shown in Fig. 2c, may be used. The EMDR equipment may be integrated with the MR device or may be positioned elsewhere and projected in the patient's view (i.e. by mirrors, lasers, etc). The EMDR device may be (co-)controlled by a processor that is in communication with the processor analyzing the fMRI data, which may directly or indirectly influence the operation of the EMDR device to adapt the BLA the patient receives.

fMRI (and MR imaging in general) is often already a difficult experience for a patient, due to the claustrophobic nature of the bore of the MR scanner and the coils, as well as the loud noises and the often extended time it takes to scan. Especially for traumatized patients this experience may even be worse and the patient may not react optimally to the EMDR treatment. Obviously this has to be taken into account when expectations are set for the patient and therapist. There are many ways known to reduce anxiety due to the scanning equipment and procedure, such as training and informing a patient, sound reduction measures and visual resources. These will not be further expounded upon here, as the skilled person would be aware of them, except to say that they may be used in combination with the presently claimed invention, as long as they don't interfere with the EMDR treatment (e.g. certain visual aids may not be applicable in this case). On the other hand visual and auditory information for increasing the patient's experience may actually be used as part of the EMDR therapy by incorporating or integrating auditory guidance and/or visual stimuli into the provided visual information.

In an embodiment of the present invention first MRI data of the patient 20 is obtained, particularly a head MRI which uses a sequence that is able to obtain a BOLD signal or another representative signal of area(s) or anatomy(/ies) of interest of the brain (hereafter simply referred to as area of interest whether is specific to one or more anatomies or areas) that may be indicative of being susceptible to be influenced by a (successful) EMDR therapy, e.g. fMRI data including a BOLD signal of the patient's amygdala 11 and/or prefrontal cortex 13. As was preciously explained, PTSD patients often have increased activation of the amygdala (caused by decreased activity of, amongst others, the prefrontal cortex), which would be identified as a high BOLD level of this anatomy. A successful treatment of PTSD therefore would be indicated by a reduced BOLD level of the amygdala. Other brain or spinal cortex anatomies (e.g. the dorsal and rostral anterior cingulate cortices, the ventromedial prefrontal cortex, the hippocampus) may be used as well for PTSD trauma level determination and therapy analysis or for various other psychological or mental traumas or neural conditions, as long as they are sufficiently indicative of a successful EMDR session. The first fMRI data is preferably obtained before the EMDR sessions commences and can as such be used as a baseline value to determine the effectiveness of the following treatment.

Second fMRI data is obtained of the same area of interest during or after the EMDR treatment and preferably also using the same sequence to obtain a comparable signal of the area of interest.

Optionally a third or even further ('later') fMRI data is obtained of the same area of interest during or after the EMDR treatment and preferably also using the same sequence to obtain a comparable signal of the area of interest. As such a comparable sequence of fMRI data of the same brain area is obtained. Preferably the first fMRI data is obtained before the EMDR therapy sessions and/or the later (preferably the last) fMRI data is obtained after the EMDR therapy session with one or more fMRI data obtained during the EMDR session to be able to compare fMRI data of the same area of interest covering the EMDR therapy session to obtain a temporal progress of the effect of the EMDR therapy on the area of interest.

The obtained fMRI data is analyzed by identifying a trauma level metric of the patient. When there is already an indication which area of interest would be most suitable to monitor for the particular trauma of the patient, then this may already be taken into account in a setting fMRI acquisition parameters beforehand. Otherwise, for instance, a larger part of the patient's brain may be imaged and the most suitable area of interest may be determined by the therapist or through an automated (for instance a machine learning) algorithm. This may be done in real-time, preferably using an algorithm, as the therapist may not be able to multitask this with optimally concentrating on the patient and performing the therapy.

Using (real-time) fMRI data provides a much better and controlled therapy than what is currently known in the field, since this information may be advantageously used by the therapist to base decisions during or after the treatment to optimize the (individually based and personalized) EMDR therapy by updating (readapting) visual stimulation.

A particularly suitable trauma metric of interest is BOLD, which is a well-studied fMRI metric and strongly indicative of local brain activity, as is described previously. It is for instance known from fMRI studies that PTSD patients have impaired extinction recall and higher amygdala activity. Also it is known that PTSD patients experience significantly increased BOLD action in the left amygdala while accomplishing happy emotion induction tasks. As such it is known how and where in a PTSD patient's brain fMRI may be employed to monitor potential changes in BOLD signal and therefore a potential effect of a treatment. It is further known that increased skin conductance occurs in PTSD subj ects

While BOLD is not easy to calibrate and/or quantify in a general manner between patients, comparing various BOLD levels over time for the same patient under the same or similar conditions provides an indication suitable for detecting change over time in the BOLD levels.

A result of a successful EMDR session is that brain activity is changed (i.e. increased or decreased) in the observed relevant anatomy or anatomies, obviously provided that the change is in the right direction. As it is possible to use fMRI in real-time or near real-time, the therapist may immediately detect a result (or lack thereof) of elements of the induced eye movements and may pursue the same or modify the BLS.

In other examples brain activity may actually be lower in certain anatomies for specific traumas and in those cases an increase of oxygenation of the anatomy may be used as an indication of a successful EMDR session. It may even occur that multiple areas of interest are affected by increased and/or decreased oxygenation.

For instance a BOLD signal of the left amygdala is obtained before, once or more during, directly after and one or more days after the EMDR treatment of a PTSD trauma patient (e.g. as the first fMRI data of a next EMDR therapy session).

Other suitable metrics that are obtainable using fMRI and that may be suitable as alternative or in addition to BOLD for certain types of areas of interest and/or certain types of trauma may be regional cerebral blood flow (CBF), cerebral blood volume (CBV), cerebral metabolic rate of oxygen (CMRO₂) or local brain conductivity (for instance as obtained by the electric properties tomography (EPT) variation of fMRI).

Furthermore, the fMRI data may be used in combination with known non-fMRI metrics such as skin conductance, electrical activity of the brain (EEG), heart rate (ECG), breath intensity, eye blinking rate, body temperature or any other physical property of the patient that could serve as an indication of effectiveness of the EMDR therapy.

Once one or more suitable trauma metrics of interest are identified then the metric is quantified for each obtained fMRI data, i.e. a BOLD level is determined and an oxygenation level is determined from this for the area of interest for each obtained fMRI data.

Next the one or more suitable trauma metrics of interest of each obtained fMRI data are compared to each other and checked if the metric has changed (i.e. increased, decreased or remained substantially the same). A difference in the metric provides a measure (e.g. a value) of the effectiveness of the EMDR therapy. A large difference may indicate a large effect of the therapy on the patient and if the difference is in the desired direction (i.e. lower or higher oxygenation of the area of interest) then a large difference indicates a strong positive response to the therapy. If the effect is small, (nearly) zero or even a difference in the wrong direction, then this is an indication that the used therapy or parameters within the therapy are not effective or may even be counter-effective.

The trauma level decrease (or lack thereof) is communicated, for instance by providing the actual values of the metric, for instance visually presented in a table or graph. Also the actual fMRI image may be provided with an indication thereon for a therapist to see. The communication could also be in the form of an auditory or tactile signal or any other manner in which a therapist would become aware of the effectiveness of the EMDR therapy.

The trauma level decrease (or lack thereof) may be presented after the treatment for the therapist to study the effectiveness in hindsight or in (semi-)real-time during the EMDR therapy, allowing the therapist to adjust the therapy if necessary or to learn which parts of the therapy are most effective and focus on those.

Alternatively the trauma level change may be communicated to another processor that automatically modifies an automated EMDR device (e.g. with pulsators 231 as shown in Fig. 2c and suitable or adapted for use in an MR environment)

In an example the difference in the trauma metric is translated into an effectiveness parameter of the EMDR therapy that is preferably easy to interpret by the therapist. The effectiveness parameter may be a value of a trauma metric (e.g. a BOLD value, blood oxygen level, etc.) or it may be a relative value (e.g. a percentage) of an initial value obtained based on the trauma metric at the first fMRI or from a previous fMRI, for instance obtained in relation to a past EMDR session. The effectiveness parameter may be communicated in the form of numericals, a graph, words or messages, a table, a color code, etc.

In an example the difference in the trauma metric may be communicated by providing actual (real-time) fMRI images (sequentially or next to each other) on which the area of interest is color-coded and a change in color indicates a change in effectiveness (e.g. shades of green for increasing effectiveness and shades of red for decreasing effectiveness). A warning signal may be given when it is detected that a change in the trauma level metric is not having an effect or an adverse effect. This may be the only communication that is provided, where the therapist receives only feedback when the therapy is not successful, which results in a low-stimulus environment for the patient and the therapist, allowing them both to optimally concentrate on the therapy itself.

When the latest or last (i.e. the second or further) fMRI data is obtained after the EMDR therapy session, this may potentially reveal lasting effects of the therapy. The second (or further) fMRI data may be obtained directly after the treatment or at a later stage for this purpose. This information may be used if further sessions are necessary and/or whether the EMDR therapy should be adapted for a next session (e.g. different auditory guidance and/or visual stimulation).

As such, two relevant tasks (meaning stimulation received by the patient and/or the trauma recollection or other specific mental or physical task the patient is requested to perform) in the EMDR procedure may be distinguished: (1) The EMDR task during therapy, when there are repetitive eye movements and (2) a general neutral/fear/startle/anxiety task before or after therapy, when there are no repetitive eye movements.

Brain activities measured during task (1) may be indicative of whether the therapy has any direct effect and the therapist could (potentially in real-time) adapt the provided BLA stimulation to it.

The brain activities measured during task (2) may be indicative of whether the EMDR therapy, in the previous or just completed session(s), has resulted in any long-lasting beneficial effects, and also this information may cause the therapist to adapt the treatment setting(s) in follow-up sessions. The absolute level of the brain activity during task (2) depends on what the exact task of the patient was: if, for instance, the patient was instructed to recall the traumatic situation (just as in EMDR, but this time without the induced eye movements), then the brain activity level might be similar in absolute level as during task (1). But if the patient was asked to just keep still, the brain activity might be less than measured during task (1), when the patient was asked to think of trauma. It is possible that the absolute levels of brain activity are different for tasks (1) and (2), even if taken quickly one after another. This may be used to determine a baseline level or to quantify the effect over time. In one example, the EMDR analysis device as claimed may autonomously communicate (or even already plan or initialize) an adaptation of the current or follow-up treatment.

In one example, the EMDR analysis device as claimed may provide, alternatively or simultaneously, another sensory modality than visual stimulation, i.e. auditory (headset for providing auditory stimulation to patient, adapting auditory signals (type, loudness, timbre, alternation frequency, etc.) or tactile stimulation (stimulator having tactile stimulation). This additional stimulation may be tuned to be compatible with the imaging device's patient experience enhancing visual and/or auditory means, i.e. by providing the stimulation in a rhythmic way, preferably in a rhythm that is compatible with (undesired) stimulation of the MR device itself.

In one example the EMDR analysis device as claimed has a learning module based on data and information collected during previously therapeutic sessions: for instance to provide automatic selection of light patterns or conditions (luminance, color, speed and sequence).

When the latest or last (i.e. the second or further) fMRI data is obtained during the EMDR therapy session, this may be used to inform the therapist during the EMDR therapy about the effectiveness of the treatment, which may cause the therapist to adjust the therapy in real-time. Or, the second (or further) fMRI data may be used to adjust settings of EMDR equipment, similar to the one shown in described in relation to Fig. 2c.

As such a patient's response to the EMDR is tracked and continued or adapted is fully personalized using only (real-time) data from the patient himself.

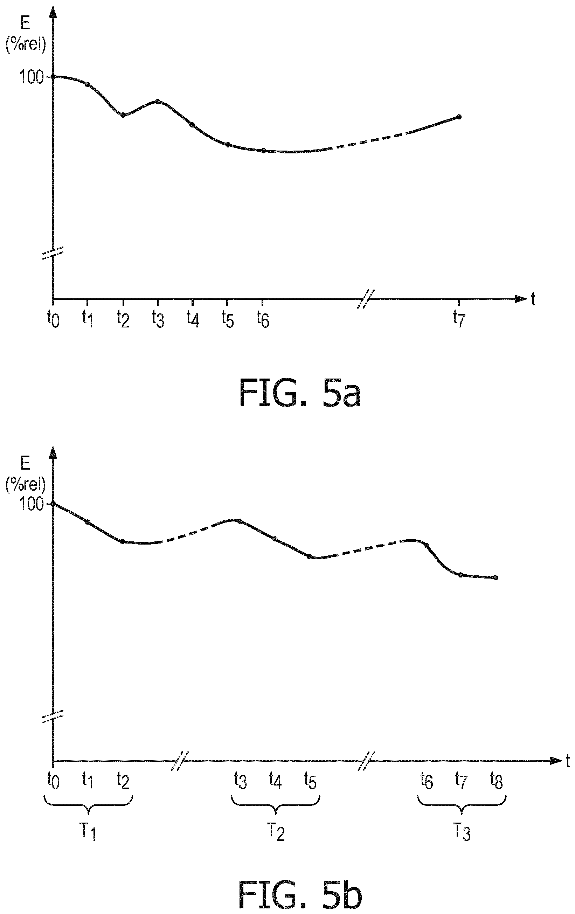

Fig. 5 depicts a simulated example of a therapy analysis output as a result of the device or method of the present invention.

In Fig. 5a a graph is shown in which an effectiveness parameter (E) is shown over a time period covering one EMDR therapy session. In this constructed, illustrative example a fictional patient having PTSD is treated and his left amygdala imaged. The trauma level metric is a BOLD value that is derived from the fMRI data. The effectiveness parameter is plotted as a relative percentage compared to an initial base value, which here corresponds with the BOLD value determined just before the EMDR therapy commenced (to). Five fMRI data were obtained during the therapy at different times (t₁, t₂, t₃, t₄, t₅). One fMRI data was obtained directly after the treatment (t₆) and one was obtained some time later (t₇), e.g. a few hours or days later. As can be seen in the graph, the EMDR was initially successful at ti and t₂, but apparently the patient reacted adversely at t₃, which was communicated to the therapist, for instance by means of showing a variation of the graph shown in Fig. 5a, a color coded message or color-enhanced 'live' fMRI image, etc. After the communication the therapist adapted the therapy, resulting in a further decrease of the effectiveness parameter E (indicating a positive result in that the trauma level has decreased). Alternatively, if the fictional patient was treated using an EMDR device for visual stimulation, e.g. similar to the one shown and described in relation to Fig. 2c, the change in the wrong direction at t₃ is communicated to a processor, which may the same processor analyzing the fMRI data, which translates this to a (proposed) adapted EMDR protocol (e.g. an adapted light sequence), for which it may or may not be necessary for a therapist to accept or deny the updated protocol.

After the therapy the BOLD level was significantly reduced, indicating that the therapy was largely successful, but over time (at t₇) it is determined that the effectiveness parameter has increased somewhat (but not to the level of the initial value, indicating a lasting effect of the therapy). The therapist may choose to schedule a further session to decrease the trauma even further.

Fig. 5b shows an illustrative, constructed example in which a fictional patient has undergone three EMDR therapy sessions (T₁, T₂, T₃) with some time between sessions (e.g. days or weeks). For each session fMRI data was obtained similar to the example of Fig. 5a, directly before, during and directly after the session. It is immediately clear that each session resulted in a decrease of the effectiveness parameter (E) and therefore reduced the trauma level. The initial effectiveness parameter was slightly increased over time, but each successive EMDR session resulted in a lowering of the trauma level and after each session the therapist may decide if the trauma has now been addressed sufficiently or if another session is required.

The presently claimed invention is directed towards EMDR treatment, but in the context of the present invention the very familiar term EMDR is considered to also cover a somewhat wider scope including closely-related and very similar BLS therapies, such as Integral Eye Movement Therapy (IEMT). The hand movement x, -x of the treatment as described and illustrated may also cover non-horizontal movement directions such as vertical or diagonal movement directions.

The presently claimed invention is illustrated mainly with fMRI of the amygdala and/or prefrontal cortex of a PTSD patient, but the invention may be adapted for other areas of interest and other neural conditions and traumas as well by a person skilled in the art who knows which part of the brain is the best indicator for trauma reduction and how this brain area should react to a successful treatment.

The term data may in the context of this invention be used in singular or plural form and should be read as all data obtained during a single imaging procedure or within a predetermined time within an extended procedure.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An Eye Movement Desensitization and Reprocessing (EMDR) therapy analysis device for analyzing an EMDR therapy for treating a patient (20) with a psychological or mental trauma, comprising
- a computer implemented system comprising:
- a computer processor configured to:
- receive first functional magnetic resonance imaging (fMRI) data from a patient before or during undergoing an EMDR treatment, wherein the first fMRI data comprises fMRI data from an area of interest in the patient's brain (10) or spinal cord (15) that is known to be associated with a trauma level of the patient;
- receive second functional magnetic resonance imaging (fMRI) data from the patient during or after undergoing an EMDR treatment, wherein the second fMRI data is obtained after the first fMRI data and comprises fMRI data from the area of interest in the patient's brain (10) or spinal cord (15) that is known to be associated with the patient's trauma;
- analyze the first fMRI data and the second fMRI data by identifying a trauma level metric indicating trauma level of the patient;
- compare the trauma level metric of the second fMRI data with the first fMRI data and determine whether the trauma level of the patient has changed;
- communicate whether the trauma level has changed.

2. An EMDR therapy analysis device according to claim 1, wherein the computer processor is further configured to:
- receive third updated fMRI data from a patient (20) during or after undergoing an EMDR treatment, wherein the third fMRI data is obtained after the second fMRI data and comprises fMRI data from the area of interest in the patient's brain (10) or spinal cord (15) that is known to be associated with the patient's trauma;
- analyze the first updated fMRI data and the second updated fMRI data by identifying a trauma level metric indicating trauma level of the patient;
- compare the trauma level metric of the third updated fMRI data with the second fMRI data and determine whether the trauma level of the patient has changed.

3. An EMDR therapy analysis device according to claim 1 or 2, wherein the computer processor is further configured to:
- receive a sequence of at least two consecutive updated fMRI data from a patient (20) during or after undergoing an EMDR treatment, wherein the fMRI data comprises fMRI data from the area of interest in the patient's brain (10) or spinal cord (15) that is known to be associated with the patient's trauma;
- analyze at least two updated fMRI data by identifying a trauma level metric indicating trauma level of the patient, wherein the at least two updated fMRI data comprises an earlier obtained MRI data and a later obtained fMRI data, which was obtained at a later stage than the earlier fMRI data, preferably the later fMRI data is the last obtained fMRI data of the sequence of consecutive fMRI data;
- compare the trauma level metric of the later fMRI data with the earlier fMRI data and determine whether the trauma level of the patient has changed.

4. An EMDR therapy analysis device according to any of the previous claims, wherein the first fMRI data is data from the patient (20) before undergoing the EMDR treatment.

5. An EMDR therapy analysis device according to claim 3 or 4, wherein the later fMRI data is data from the patient (20) after undergoing the EMDR treatment.

6. An EMDR therapy analysis device according to any of the previous claims, wherein the trauma metric of interest is a blood oxygenation level dependent (BOLD) contrast.

7. An EMDR therapy analysis device according to any of the previous claims, wherein the trauma is post-traumatic stress disorder (PTSD) and the area of interest in the patient's brain preferably comprises the patient's amygdala (11), preferably the patient's left amygdala, and/or the patient's prefrontal cortices (13, 13-1, 13-2).

8. An EMDR therapy analysis device according to any of the previous claims, wherein the trauma level is further determined by combining received EMDR data with other physiologic or biometric data of the patient, for instance skin conductance, electrical activity of the brain, heart rate, breath intensity, eye blinking rate, body temperature or any other physical property of the patient that could serve as an indication of effectiveness and/or other fMRI or fMRI-like data, such as regional cerebral blood flow (CBF), cerebral blood volume (CBV), cerebral metabolic rate of oxygen (CMRO₂) or local brain conductivity (for instance as obtained by electric properties tomography).

9. An EMDR therapy analysis device according to any of the previous claims, wherein the computer processor is configured to communicate whether the trauma level has changed to a communication device that is available to an EMDR therapist during a therapy, for instance by displaying visual information or through auditory or tactile signals, preferably during or shortly after a EMDR therapy session.

10. An EMDR therapy analysis device according to any of the previous claims, wherein the computer processor is configured to communicate whether the trauma level has changed to a device that is capable of adapting the EMDR therapy during an ongoing EMDR therapy session, such as a device for generating adaptive visual stimulation, such as an MDR device comprising a light tube (23) and/or pulsators (231), and/or providing a suggestion or plan for a future EMDR session for the patient.

11. An EMDR therapy analysis device according to any of the previous claims, wherein the processor is further configured to translate a trauma level metric change into an effectiveness parameter, for instance a known value of the trauma level metric and/or a relative value compared to an initial value, for instance a relative value, e.g. a percentage, of the value of the trauma level metric of the first fMRI data; and wherein the communication device is further configured to communicate the effectiveness parameter.

12. An EMDR therapy analysis method comprising the steps of:
- obtaining first functional magnetic resonance imaging (fMRI) data from a patient (20) before or during undergoing an EMDR treatment, wherein the first fMRI data comprises fMRI data from an area of interest in the patient's brain (10) or spinal cord (15) that is known to be associated with a trauma level of the patient;
- obtaining second functional magnetic resonance imaging (fMRI) data from the patient (20) during or after undergoing an EMDR treatment, wherein the second fMRI data is obtained after the first fMRI data and comprises fMRI data from the area of interest in the patient's brain (10) or spinal cord (15) that is known to be associated with the patient's trauma;
- analyzing the first fMRI data and the second fMRI data by identifying a trauma level metric indicating trauma level of the patient;
- comparing the trauma level metric of the second fMRI data with the first fMRI data
- determining whether the trauma level of the patient has changed;
- communicating whether the trauma level has changed.

13. An EMDR therapy analysis method according to claim 12 comprising the further steps of:
- receiving a sequence of consecutive updated fMRI data from a patient (20) during or after undergoing an EMDR treatment, wherein the fMRI data comprises fMRI data from the area of interest in the patient's brain (10) or spinal cord (15) that is known to be associated with the patient's trauma;
- analyzing at least two updated fMRI data by identifying a trauma level metric indicating trauma level of the patient, wherein the at least two updated fMRI data comprises an earlier obtained MRI data and a later obtained fMRI data, which was obtained at a later stage than the earlier fMRI data, preferably the later fMRI data is the last obtained fMRI data of the sequence of consecutive fMRI data;
- comparing the trauma level metric of the later fMRI data with the earlier fMRI data and determine whether the trauma level of the patient has changed.

14. An EMDR therapy analysis method according to any of claim 12 or 13, wherein the trauma is post-traumatic stress disorder (PTSD) and the area of interest in the patient's brain preferably comprises the patient's amygdala (11), more preferably the patient's left amygdala, and/or the patient's prefrontal cortices (13, 13-1, 13-2) and wherein the trauma level metric is preferably a blood oxygenation level dependent (BOLD) contrast.

15. A computer program product configured to, when running, to execute the steps of the any of the methods of claim 12-14.
